# EUROPEAN PATENT APPLICATION

(11) **EP 4 674 356 A1**
(43) Date of publication of application: **07.01.2026**
(21) Application number: 24763670.7
(22) Date of filing: 19.02.2024
(51) Int. Cl.: A61B 8/08

(54) **ULTRASOUND DIAGNOSTIC DEVICE AND CONTROL METHOD FOR ULTRASOUND DIAGNOSTIC DEVICE**

(30) Priority: 02.03.2023 JP 2023031767
(71) Applicant: FUJIFILM Corporation, Tokyo 106-8620 (JP)
(72) Inventor: MARUO Yusuke, Tokyo 106-8620 (JP); SATO Ryosuke, Tokyo 106-8620 (JP)
(74) Representative: Klunker IP Patentanwälte PartG mbB
(86) International application number: PCT/JP2024/005661
(87) International publication number: WO 2024/181188

(57) **Abstract**

An ultrasonic diagnostic apparatus includes: an image acquisition unit (24) that acquires an ultrasonic image in which a mammary gland region of a subject is imaged; a monitor (23) that displays the ultrasonic image; a mammary gland region extraction unit (25) that extracts the mammary gland region from the ultrasonic image; an evaluation unit (27) that performs a glandular tissue component evaluation based on the mammary gland region; a schematic diagram creation unit (30) that creates a breast schematic diagram showing a breast divided into a plurality of partial regions; and a display control unit (22) that displays an evaluation result of the glandular tissue component evaluation on the monitor (23) in association with a partial region, which corresponds to a position where the ultrasonic image is captured, among the plurality of partial regions in the breast schematic diagram.

## Description

### BACKGROUND OF THE INVENTION

### 1. Field of the Invention

The present invention relates to an ultrasonic diagnostic apparatus used for an examination of a breast of a subject and a method of controlling an ultrasonic diagnostic apparatus.

### 2. Description of the Related Art

In related art, in the medical field, an ultrasonic diagnostic apparatus using ultrasonic images is put into practical use. In general, the ultrasonic diagnostic apparatus comprises an ultrasonic probe provided with a transducer array and an apparatus body connected to the ultrasonic probe, in which an ultrasonic beam is transmitted from the ultrasonic probe toward a subject, an ultrasonic echo from the subject is received by the ultrasonic probe, and a reception signal is electrically processed to generate the ultrasonic image.

A composition of a fat tissue and a mammary gland tissue in a breast varies depending on a person, but an anatomical structure of the breast is common, and a primary lactiferous duct branches into extralobular ducts, which in turn connect to numerous lobules, in the mammary gland tissue. Stroma is present around the lobules, and mammary gland tissue is composed of the lobules together with the stroma.

It is known that two types of stroma exist around the lobules, that is, perilobular stroma and edematous stroma. The perilobular stroma exists along a structure from the lobule to the mammary duct, and includes many collagen fibers. On the other hand, the edematous stroma fills the spaces between the perilobular stroma, is rich in extracellular matrix, with a mixture of collagen fibers and fat, and contains fewer collagen fibers as compared to the perilobular stroma.

In recent years, the concept of individualized risk management for patients has become widespread, but it is known that a ratio of the mammary gland region within the breast, especially a high-density mammary gland, is a risk factor for cancer. The ratio of the mammary gland region in the breast can be measured by using a mammography apparatus.

Further, in Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case in which a ratio of a glandular tissue component (GTC) region including mammary ducts, lobules, and perilobular stroma in the mammary gland region is high even though the mammary gland region is almost the same. Stated another way, a ratio of the GTC region in the mammary gland region may be a risk factor, in addition to the ratio of the mammary gland region in the breast. This means that a patient with less advanced atrophy of the lobule has a higher risk.

However, in the mammography apparatus, the perilobular stroma and the edematous stroma cannot be distinguished from each other, and the entire mammary gland tissue is observed as whitish, and as a result, the ratio of the GTC region in the mammary gland region cannot be measured.

WO2018/180386A discloses an ultrasonic diagnostic apparatus that detects a lesion in a mammary gland region.

### SUMMARY OF THE INVENTION

However, an object of the ultrasonic diagnostic apparatus disclosed in WO2018/180386A is to detect the lesion part in the mammary gland region, and is not concerned with further subdividing the mammary gland region into smaller regions. Therefore, there is an issue in that the risk of cancer in the mammary gland region cannot be considered in detail.

In addition, in order to further consider the risk of cancer in the mammary gland region in more detail, it is desirable to divide the breast into a plurality of regions and give consideration for each divided region.

The present invention has been made in order to solve such an issue in the related art, and an object of the present invention is to provide an ultrasonic diagnostic apparatus that enables a user to consider a risk of cancer in a mammary gland region of a subject in detail.

It is possible to achieve the above-described object with the following configurations.
[1] An ultrasonic diagnostic apparatus comprising: an image acquisition unit that acquires an ultrasonic image in which a mammary gland region of a subject is imaged; a monitor that displays the ultrasonic image; a mammary gland region extraction unit that extracts the mammary gland region from the ultrasonic image; an evaluation unit that performs a glandular tissue component evaluation based on the mammary gland region extracted by the mammary gland region extraction unit; a schematic diagram creation unit that creates a breast schematic diagram showing a breast divided into a plurality of partial regions; and a display control unit that displays an evaluation result of the glandular tissue component evaluation performed by the evaluation unit on the monitor in association with a partial region, which corresponds to a position where the ultrasonic image is captured, among the plurality of partial regions in the breast schematic diagram.
[2] The ultrasonic diagnostic apparatus according to [1], further comprising: a glandular tissue component region extraction unit that extracts a glandular tissue component region from the mammary gland region extracted by the mammary gland region extraction unit, in which the evaluation unit performs the glandular tissue component evaluation based on a ratio of the glandular tissue component region in the mammary gland region.
[3] The ultrasonic diagnostic apparatus according to [2], in which the evaluation unit displays the ratio of the glandular tissue component region in the mammary gland region on the monitor as the evaluation result.
[4] The ultrasonic diagnostic apparatus according to [2], in which the evaluation unit determines a category of the glandular tissue component region based on the ratio of the glandular tissue component region in the mammary gland region, and displays the category on the monitor as the evaluation result.
[5] The ultrasonic diagnostic apparatus according to [1], in which the evaluation unit performs the glandular tissue component evaluation using a determination model that has been trained through machine learning.
[6] The ultrasonic diagnostic apparatus according to any one of [1] to [5], in which the image acquisition unit acquires a plurality of the ultrasonic images at positions corresponding to the same partial region, and the evaluation unit determines the evaluation result in the partial region based on a plurality of the evaluation results acquired by performing the glandular tissue component evaluation on each of the plurality of ultrasonic images.
[7] The ultrasonic diagnostic apparatus according to [6], in which the evaluation unit determines the evaluation result for a specific ultrasonic image by taking into account the plurality of evaluation results in the ultrasonic images of a predetermined number of frames immediately before the specific ultrasonic image.
[8] The ultrasonic diagnostic apparatus according to any one of [2] to [4], in which the glandular tissue component region extraction unit extracts the glandular tissue component region only for the ultrasonic image from which the mammary gland region is extracted by the mammary gland region extraction unit.
[9] The ultrasonic diagnostic apparatus according to any one of [1] to [8], further comprising: a notification unit that issues notification to a user in a case in which the evaluation result of the glandular tissue component evaluation performed by the evaluation unit exceeds a predetermined threshold value.
[10] The ultrasonic diagnostic apparatus according to [9], in which the notification unit displays a position where the ultrasonic image in which the evaluation result exceeds the threshold value is captured, on the breast schematic diagram.
[11] The ultrasonic diagnostic apparatus according to [9] or [10], in which the notification unit highlights a specific portion of a display screen of the monitor.
[12] A method of controlling an ultrasonic diagnostic apparatus, the method comprising: acquiring an ultrasonic image in which a mammary gland region of a subject is imaged; displaying the ultrasonic image on a monitor; extracting the mammary gland region from the ultrasonic image; extracting a glandular tissue component region from the extracted mammary gland region; performing a glandular tissue component evaluation based on a ratio of the glandular tissue component region in the mammary gland region; creating a breast schematic diagram showing a breast divided into a plurality of partial regions; and displaying an evaluation result of the glandular tissue component evaluation on the monitor in association with a partial region, which corresponds to a position where the ultrasonic image is captured, among the plurality of partial regions in the breast schematic diagram.

According to the aspects of the present invention, the ultrasonic diagnostic apparatus comprises: the image acquisition unit that acquires the ultrasonic image in which the mammary gland region of the subject is imaged; the monitor that displays the ultrasonic image; the mammary gland region extraction unit that extracts the mammary gland region from the ultrasonic image; the evaluation unit that performs the glandular tissue component evaluation based on the mammary gland region extracted by the mammary gland region extraction unit; the schematic diagram creation unit that creates the breast schematic diagram showing the breast divided into the plurality of partial regions; and the display control unit that displays the evaluation result of the glandular tissue component evaluation performed by the evaluation unit on the monitor in association with the partial region, which corresponds to a position where the ultrasonic image is captured, among the plurality of partial regions in the breast schematic diagram, so that the user can consider a risk of cancer in the mammary gland region of the subject in detail.

### BRIEF DESCRIPTION OF THE DRAWINGS

Fig. 1 is a block diagram showing a configuration of an ultrasonic diagnostic apparatus according to an embodiment of the present invention.
Fig. 2 is a block diagram showing an internal configuration of a transmission-and-reception circuit according to the embodiment.
Fig. 3 is a block diagram showing an internal configuration of an image generation unit according to the embodiment.
Fig. 4 is a diagram showing an ultrasonic image obtained by imaging a mammary gland region of a subject.
Fig. 5 is a diagram showing an ultrasonic image including a mammary gland region in which a GTC region is imaged.
Fig. 6 is a diagram showing a first example of a breast schematic diagram.
Fig. 7 is a diagram showing a second example of the breast schematic diagram.
Fig. 8 is a diagram showing a third example of the breast schematic diagram.
Fig. 9 is a diagram showing a fourth example of the breast schematic diagram.
Fig. 10 is a diagram showing a fifth example of the breast schematic diagram.
Fig. 11 is a diagram showing a sixth example of the breast schematic diagram.
Fig. 12 is a diagram showing a seventh example of the breast schematic diagram.
Fig. 13 is a diagram showing an eighth example of the breast schematic diagram.
Fig. 14 is a diagram showing a display example of an evaluation result on a monitor.
Fig. 15 is a diagram showing a first display example of the evaluation result on the breast schematic diagram.
Fig. 16 is a diagram showing a second display example of the evaluation result on the breast schematic diagram.
Fig. 17 is a diagram showing a third display example of the evaluation result on the breast schematic diagram.
Fig. 18 is a diagram showing a fourth display example of the evaluation result on the breast schematic diagram.
Fig. 19 is a diagram showing a first example of notification.
Fig. 20 is a diagram showing a second example of the notification.
Fig. 21 is a diagram showing a third example of the notification.
Fig. 22 is a flowchart showing an operation according to the embodiment.
Fig. 23 is a diagram showing a fifth display example of the evaluation result on the breast schematic diagram.
Fig. 24 is a diagram showing a sixth display example of the evaluation result on the breast schematic diagram.
Fig. 25 is a diagram showing a seventh display example of the evaluation result on the breast schematic diagram.

### DESCRIPTION OF THE PREFERRED EMBODIMENTS

Hereinafter, embodiments of the invention will be described with reference to the accompanying drawings.

The following configuration requirements are described based on a representative embodiment of the present invention, but the present invention is not limited to such an embodiment.

In the present specification, a numerical range represented by "to" means a range including numerical values described before and after "to", both ends inclusive, as a lower limit value and an upper limit value.

In the present specification, "same" and "identical" include an error range that is generally allowed in the technical field.

### [Embodiment]

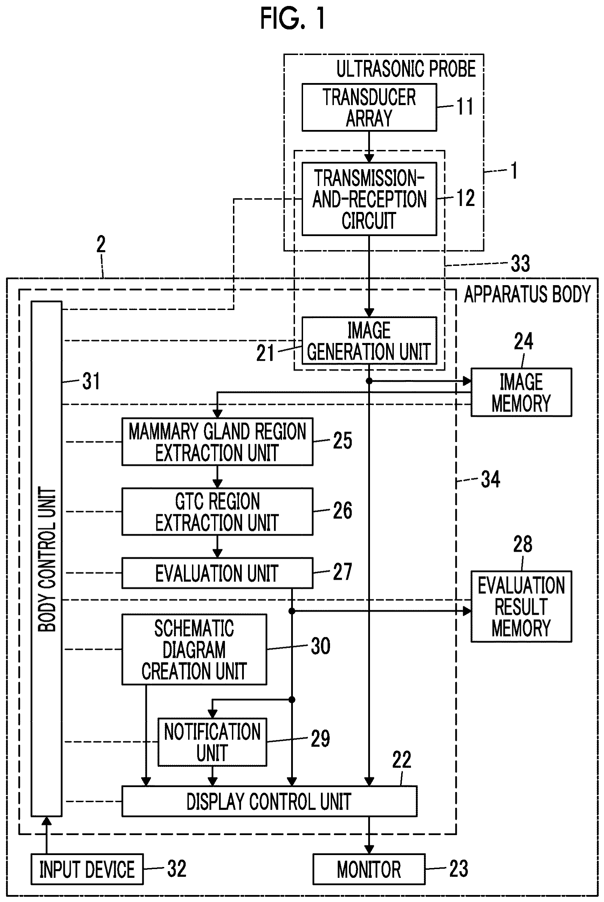

Fig. 1 shows a configuration of an ultrasonic diagnostic apparatus according to the embodiment of the present invention. The ultrasonic diagnostic apparatus comprises an ultrasonic probe 1 and an apparatus body 2. The ultrasonic probe 1 and the apparatus body 2 are wired-connected to each other via a cable (not shown).

The ultrasonic probe 1 includes a transducer array 11 and a transmission-and-reception circuit 12 connected to the transducer array 11.

The apparatus body 2 includes an image generation unit 21 connected to the transmission-and-reception circuit 12 of the ultrasonic probe 1, a display control unit 22 and a monitor 23 are connected sequentially to the image generation unit 21, and an image memory 24 is connected to the image generation unit 21. In addition, a mammary gland region extraction unit 25, a glandular tissue component (GTC) region extraction unit 26, and an evaluation unit 27 are connected sequentially to the image memory 24. The evaluation unit 27 is connected to the display control unit 22. Further, an evaluation result memory 28 and a notification unit 29 are connected to the evaluation unit 27. The notification unit 29 is connected to the display control unit 22. In addition, the apparatus body 2 comprises a schematic diagram creation unit 30. The schematic diagram creation unit 30 is connected to the display control unit 22.

In addition, a body control unit 31 is connected to the transmission-and-reception circuit 12, the image generation unit 21, the display control unit 22, the image memory 24, the mammary gland region extraction unit 25, the GTC region extraction unit 26, the evaluation unit 27, the evaluation result memory 28, the notification unit 29, and the schematic diagram creation unit 30. An input device 32 is connected to the body control unit 31. The transmission-and-reception circuit 12 and the image generation unit 21 constitute an image acquisition unit 33. In addition, the input device 32 is connected to the body control unit 31. The image generation unit 21, the display control unit 22, the mammary gland region extraction unit 25, the GTC region extraction unit 26, the evaluation unit 27, the notification unit 29, the schematic diagram creation unit 30, and the body control unit 31 constitute a processor 34 for the apparatus body 2.

The transducer array 11 of the ultrasonic probe 1 includes a plurality of ultrasonic transducers arranged in a one-dimensional or two-dimensional manner. Each of these transducers transmits an ultrasonic wave in response to a drive signal supplied from the transmission-and-reception circuit 12, receives a reflected wave from a subject, and outputs an analog reception signal. Each transducer is formed by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by a lead magnesium niobate-lead titanate (PMN-PT) solid solution.

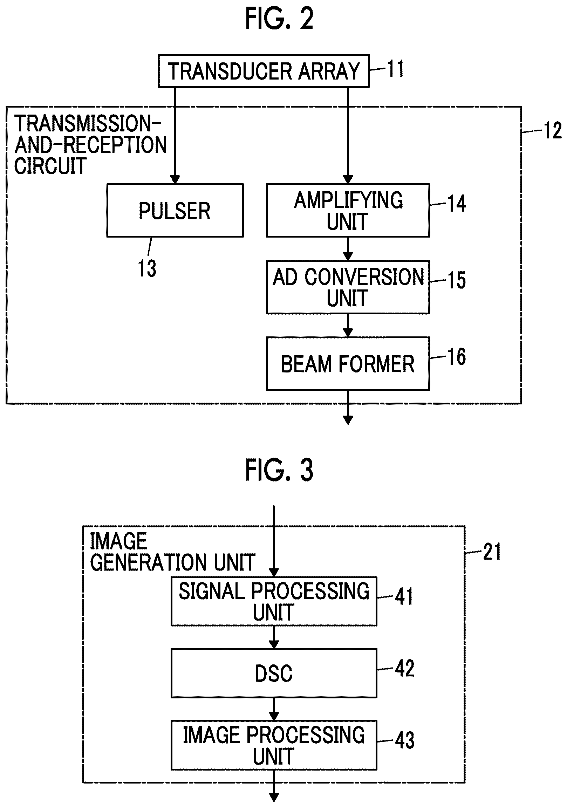

The transmission-and-reception circuit 12 transmits the ultrasonic wave from the transducer array 11 and generates a sound ray signal based on the reception signal acquired by the transducer array 11, under the control of the body control unit 31. The transmission-and-reception circuit 12 includes, as shown in Fig. 2, a pulser 13 connected to the transducer array 11, and an amplifying unit 14, an analog-to-digital (AD) conversion unit 15, and a beam former 16 which are sequentially connected in series to the transducer array 11.

The pulser 13 includes, for example, a plurality of pulse generators, adjusts a delay amount of each drive signal based on a transmission delay pattern selected in accordance with a control signal from the body control unit 31 such that ultrasonic waves to be transmitted from the plurality of transducers of the transducer array 11 form a ultrasonic beam, and supplies the drive signal of which the delay amount has been adjusted, to the plurality of transducers. In this way, in a case in which a pulsed or continuous wave voltage is applied to the electrodes of the transducers of the transducer array 11, the piezoelectric body expands and contracts to generate a pulsed or continuous wave ultrasonic wave from each transducer, and the ultrasonic beam is formed from the combined wave of these ultrasonic waves.

The transmitted ultrasonic beam is reflected by a target, for example, a part of the subject, and an ultrasonic echo propagates toward the transducer array 11 of the ultrasonic probe 1. The ultrasonic echo propagating toward the transducer array 11 in this manner is received by each of the transducers constituting the transducer array 11. In this case, each transducer constituting the transducer array 11 expands and contracts by receiving the propagating ultrasonic echo to generate the reception signal that is an electric signal, and outputs the reception signal to the amplifying unit 14.

The amplifying unit 14 amplifies the signal input from each of the transducers constituting the transducer array 11 and transmits the amplified signal to the AD conversion unit 15. The AD conversion unit 15 converts the signal transmitted from the amplifying unit 14 into digital reception data, and transmits the reception data to the beam former 16. The beam former 16 performs so-called reception focus processing by giving and adding delay with respect to each reception data converted by the AD conversion unit 15, in accordance with a sound velocity or a sound velocity distribution set based on a reception delay pattern selected according to a control signal from the body control unit 31. Through the reception focus processing, a sound ray signal is acquired in which each piece of the reception data converted by the AD conversion unit 15 is phased and added and the focus of the ultrasonic echo is narrowed.

The image generation unit 21 of the apparatus body 2 has, as shown in Fig. 3, a configuration in which a signal processing unit 41, a digital scan converter (DSC) 42, and an image processing unit 43 are sequentially connected in series.

The signal processing unit 41 performs, on the sound ray signal transmitted from the transmission-and-reception circuit 12 of the ultrasonic probe 1, correction of attenuation caused by a distance in accordance with a depth of a reflection position of the ultrasonic wave and then performs envelope detection processing, and thereby generates an ultrasonic image signal (B-mode image signal), which is tomographic image information related to tissues in the subject.

The DSC 42 converts (raster-converts) the ultrasonic image signal generated by the signal processing unit 41 into an image signal in accordance with a normal television signal scanning method.

The image processing unit 43 performs various types of necessary image processing, such as gradation processing, on the ultrasonic image signal input from the DSC 42, and then outputs the signal representing the ultrasonic image to the display control unit 22 and the image memory 24. The signal representing the ultrasonic image generated by the image generation unit 21 in this way will be simply referred to as the ultrasonic image. In addition, the image generation unit 21 can also output the ultrasonic image signal before being processed by the DSC 42 or the ultrasonic image signal immediately after being processed by the DSC 42 to the image memory 24. In this case, the image generation unit 21 can generate the ultrasonic image by reading out these signals from the image memory 24 and performing processing using the DSC 42 or the image processing unit 43.

The image memory 24 is a memory that stores the ultrasonic image generated by the image generation unit 21 under the control of the body control unit 31. For example, the image memory 24 can store a plurality of frames of ultrasonic images generated by the image generation unit 21 in correspondence with diagnosis on a mammary gland region of a breast of the subject.

As the image memory 24, for example, a recording medium such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), can be used.

The mammary gland region extraction unit 25 detects a breast region of the subject from the ultrasonic image read out from the image memory 24, and extracts the mammary gland region from the detected breast region.

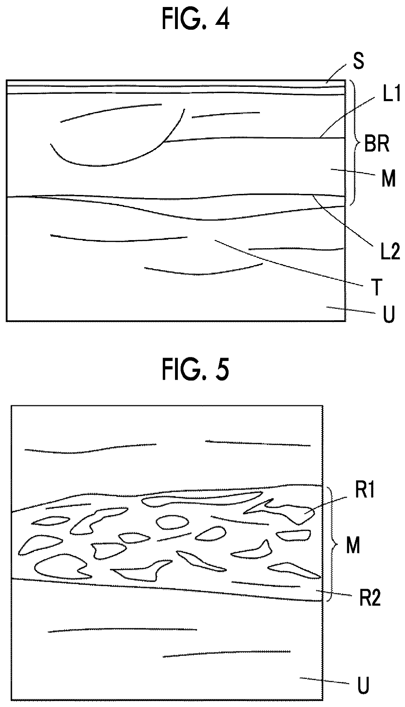

Fig. 4 shows an example of an ultrasonic image U in which the breast of the subject is imaged. The ultrasonic image U is a tomographic image captured by bringing a distal end of the ultrasonic probe 1 into contact with the breast of the subject, in which a skin S of the subject is shown in an upper end of the ultrasonic image U representing a shallowest portion, and a pectoralis major T is shown in a lower portion of the ultrasonic image U representing a deeper portion. The mammary gland region extraction unit 25 can recognize a skin S and a pectoralis major T from the ultrasonic image U and detect a deep region between the skin S and the pectoralis major T as a breast region BR.

As shown in Fig. 4, the mammary gland region extraction unit 25 can recognize a front boundary line L1 located on a shallower side and a rear boundary line L2 located on a deeper side in the detected breast region BR, and can extract a deep region between the front boundary line L1 and the rear boundary line L2 as a mammary gland region M.

In order to detect the breast region BR and to extract the mammary gland region M described above, the mammary gland region extraction unit 25 can perform image recognition using at least one of template matching, an image analysis technique using a feature value, such as adaptive boosting (AdaBoost), support vector machine (SVM), or scale-invariant feature transform (SIFT), or a determination model that has been trained by using a machine learning technique such as deep learning.

The determination model is a trained model that has learned the breast region BR and the mammary gland region M (segmentation) of the breast region BR in a training ultrasonic image obtained by imaging the breast.

As shown in Fig. 5, the GTC region extraction unit 26 extracts a GTC region R1 from the mammary gland region M of the subject extracted by the mammary gland region extraction unit 25. The GTC region R1 consists of mammary ducts, lobules, and perilobular stroma in the mammary gland region M, and edematous stroma R2 fills a space between the perilobular stroma. Since the edematous stroma R2 is rich in extracellular matrix and has a mixture of adipocytes, in a case in which the mammary gland region M is observed in the ultrasonic image U, the edematous stroma R2 has a high echo level and is shown with high brightness. On the other hand, the mammary ducts, the lobules, and the perilobular stroma constituting the GTC region R1 have relatively low echo levels, and the brightness is lower than that of the edematous stroma R2.

Therefore, the GTC region extraction unit 26 can distinguish between the GTC region R1 and the edematous stroma R2 in the mammary gland region M by, for example, binarizing the mammary gland region M of the ultrasonic image U using a brightness threshold value Thb, and can extract the GTC region R1.

In addition, a predetermined constant value can be used as the brightness threshold value Thb.

In addition, the GTC region extraction unit 26 may perform edge detection on the GTC region R1 of the ultrasonic image U by image analysis, and automatically calculate the brightness threshold value Thb based on a change in brightness values of the detected edge portion, that is, a change in brightness values of a plurality of pixels from the inside to the outside of the GTC region R1. In this way, it is possible to automatically set the brightness threshold value Thb suitable for the ultrasonic image as the image analysis target, and to acquire the binarized image suitable for the ultrasonic image U.

Further, the ultrasonic diagnostic apparatus can be configured such that a histogram of the brightness of the mammary gland region M detected from the ultrasonic image U is created, and the user sets the brightness threshold value Thb by inputting the brightness threshold value Thb from the input device 32 based on the histogram, a binarized image created using the initial value of the brightness threshold value Thb, and the ultrasonic image U generated by the image generation unit 21.

In addition, the GTC region extraction unit 26 can also extract the GTC region R1 by using a determination model that has been trained learned using a machine learning technique such as deep learning. In this case, for example, a trained model that has learned the GTC region R1 (segmentation) in the mammary gland region M in the training ultrasonic image in which the breast is imaged is used as the determination model.

The evaluation unit 27 calculates a ratio of the GTC region R1 extracted by the GTC region extraction unit 26 in the mammary gland region M extracted by the mammary gland region extraction unit 25 and performs the GTC evaluation based on the calculated ratio of the GTC region R1. In such a case, the evaluation unit 27 can divide the breast of the subject into a plurality of partial regions and perform the GTC evaluation for each of the divided partial regions.

The evaluation unit 27 can calculate a GTC region ratio, for example, by a ratio of the sum of the number of pixels occupied by all the GTC regions R1 present in the mammary gland region M to the number of pixels occupied by the mammary gland region M in the ultrasonic image U. The evaluation unit 27 can use, for example, the ratio of the GTC region R1 calculated as described above as the evaluation result.

For example, the evaluation unit 27 can determine a category of the GTC region R1 based on the ratio of the GTC region R1 in the mammary gland region M and use the category as the evaluation result.

It is generally known that the lobule atrophies with age, but there are research results that a risk of breast cancer is high in patients in whom the lobule does not atrophy, as disclosed in, for example, "Su Hyun Lee et al. "Glandular Tissue Component and Breast Cancer Risk in Mammographically Dense Breasts at Screening Breast US", Radiology, Volume 301, October 1,2021". The category of the GTC region R1 represents a degree of progression of the atrophy of the lobule, and can be used as a material for determining the risk of breast cancer.

The evaluation unit 27 can also determine the category of the GTC region R1 using, for example, a trained model that has been trained through machine learning based on a plurality of training data each of which includes the ultrasonic image U in which the breast is imaged and the category of the GTC region R1 in the breast, as shown in Figs. 4 and 5. The association between the ultrasonic image U and the category of the GTC region R1 in the training data can be performed by an expert, such as a skilled doctor.

The evaluation unit 27 can output, as the evaluation result, any one of a plurality of predetermined categories, for example, any one of two categories of Low and High as the category of the GTC region R1. Low indicates that the lobule atrophy has not progressed as much as in High. Further, the evaluation unit 27 can also output, for example, any one of four categories of Minimal, Mild, Moderate, and Marked as the category of the GTC region R1. Mild indicates that the atrophy of the lobule is not more advanced than that in Minimal, Moderate indicates that the atrophy of the lobule is not more advanced than that in Mild, and Marked indicates that the atrophy of the lobule is not more advanced than that in Moderate.

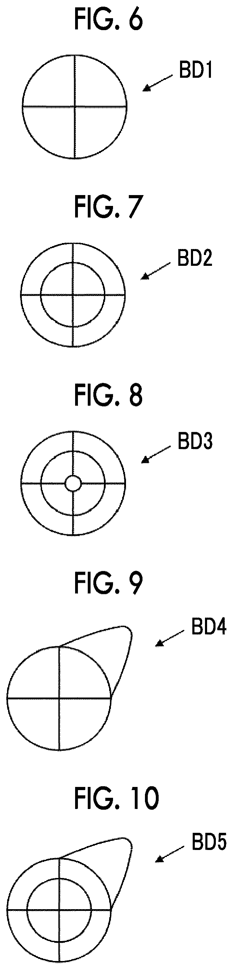

For example, as shown in Fig. 6, the schematic diagram creation unit 30 creates a breast schematic diagram BD1 representing the breast divided into the plurality of partial regions. The breast schematic diagram BD1 shown in Fig. 6 represents a right or left breast of the subject as viewed from the front, and has four partial regions of an upper right, a lower right, an upper left, and a lower left.

The schematic diagram creation unit 30 can also create the breast schematic diagrams of any format, for example, breast schematic diagrams BD2 to BD8 as shown in Figs. 7 to 13, in addition to the breast schematic diagram BD1 shown in Fig. 6. The breast schematic diagram BD2 shown in Fig. 7 represents a right or left breast of the subject, and has a total of eight partial regions by further dividing the upper right, the lower right, the upper left, and the lower left into two parts, respectively. The breast schematic diagram BD3 shown in Fig. 8 has a central circular partial region corresponding to the nipple, in addition to the eight partial regions shown in Fig. 7.

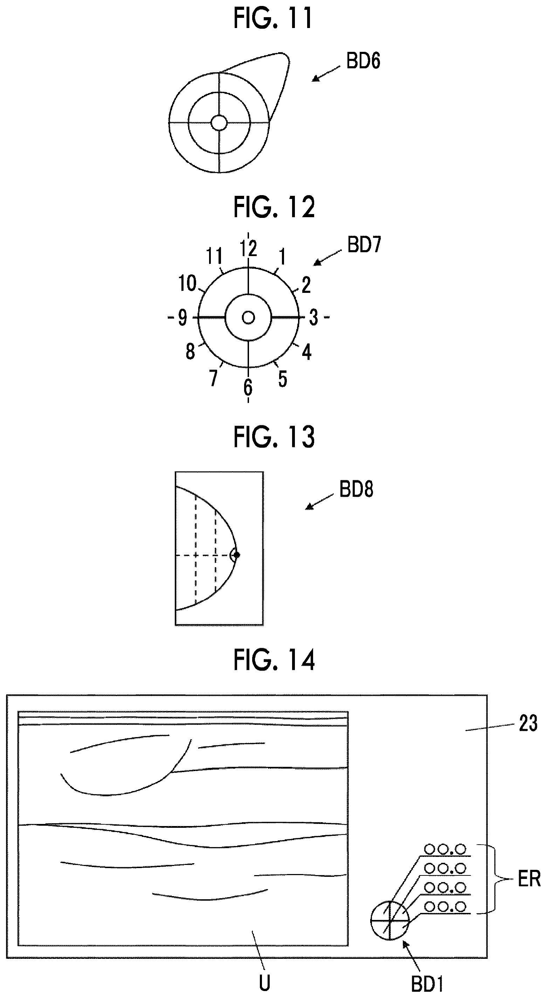

Breast schematic diagrams BD4 to BD6 shown in Figs. 9 to 11 each have a protrusion portion representing a left armpit of the subject, which extends from the breast schematic diagrams BD1 to BD3 shown in Figs. 6 to 8 toward the upper right, and represent the left breast of the subject. A breast schematic diagram BD7 shown in Fig. 12 is a derivative of the breast schematic diagram BD3 shown in Fig. 8 and the breast schematic diagram BD6 shown in Fig. 11, and only a region surrounded by a second largest circle and the largest circle among the three concentric circles is divided into four regions of the upper right, the lower right, the upper left, and the lower left, and thus a total of six partial regions are provided. In addition, in the breast schematic diagram BD7, as in a dial plate of a clock, numbers "1" to "12" are arranged corresponding to the azimuth from the center of the breast schematic diagram BD7. A breast schematic diagram BD8 shown in Fig. 13 shows the breast of the subject as viewed from the side, and has six partial regions divided by a linear center line passing through the nipple and two straight lines orthogonal to the center line.

In addition, the breast schematic diagram created by the schematic diagram creation unit 30 can be divided into any number of partial regions.

The display control unit 22 performs predetermined processing on the ultrasonic image U transmitted from the image generation unit 21 under the control of the body control unit 31, and displays the ultrasonic image U on the monitor 23. In addition, for example, as shown in Fig. 14, the display control unit 22 displays an evaluation result ER of the GTC evaluation performed by the evaluation unit 27 on the monitor 23 in association with the partial region corresponding to the position where the ultrasonic image U is captured among the plurality of partial regions in the breast schematic diagram BD1 created by the schematic diagram creation unit 30. In the example of Fig. 14, the ultrasonic image U is captured at the positions corresponding to all the four partial regions of the breast schematic diagram BD1, and the evaluation result ER is displayed in association with each of the four partial regions.

Which of the plurality of partial regions of the breast schematic diagram BD1 corresponds to the position to be imaged can, for example, be designated by the user via the input device 32 during examination of the subject's breast. For example, in a case in which the ultrasonic image U is captured in a state in which one of the plurality of partial regions is designated by the user, the partial region designated by the user is associated with the ultrasonic image U, and the ultrasonic image U and the information on the partial region, which correspond to each other, are stored in the image memory 24. The display control unit 22 reads out the information on the partial region from the image memory 24, and displays the evaluation result ER output based on the corresponding ultrasonic image U in association with the partial region.

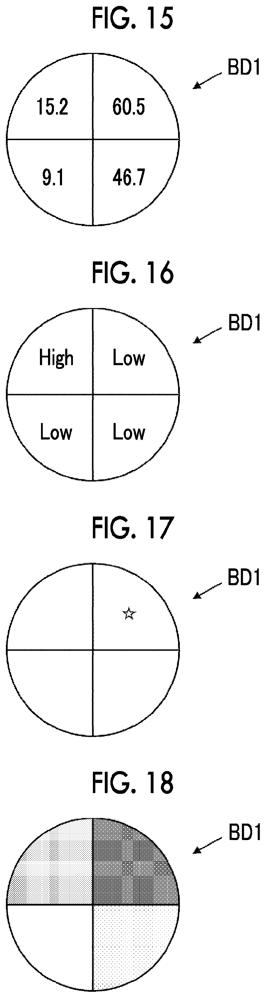

For example, as shown in Fig. 15, the display control unit 22 can display a numerical value of the ratio of the GTC region R1 in the mammary gland region M in association with the plurality of partial regions in the breast schematic diagram BD1. In addition, for example, as shown in Fig. 16, the display control unit 22 can also display the category of the GTC region R1 in the plurality of partial regions in the breast schematic diagram BD1. In the example of Fig. 16, the category of Low or High is displayed in each of the four partial regions.

In addition, the display control unit 22 can also display the evaluation result ER of the GTC evaluation in association with the partial region by, for example, providing a predetermined threshold value for the ratio of the GTC region R1 in the mammary gland region M and disposing a mark only to the partial region in which the ratio of the GTC region R1 exceeds the threshold value as shown in Fig. 17. In addition, for example, as shown in Fig. 18, the display control unit 22 can also display the evaluation result ER of the GTC evaluation by displaying the partial region in a color corresponding to the category of the GTC region R1.

Here, for example, in "Su Hyun Lee et al. "Glandular Tissue Component and Breast Cancer Risk in Mammographically Dense Breasts at Screening Breast US", Radiology, Volume 301, October 1, 2021", it is reported that cancer is likely to occur in a case in which the ratio of the glandular tissue component (GTC) region including the mammary ducts, the lobules, and the perilobular stroma in the mammary gland region M is high even though the mammary gland region M is almost the same. Stated another way, in addition to the ratio of the mammary gland region M in the breast, the ratio of the GTC region R1 in the mammary gland region M can be a risk factor. The user can confirm the evaluation result ER of the GTC evaluation displayed in association with the partial region of the breast schematic diagram BD1, to consider the risk of cancer in detail for each partial region.

The evaluation result memory 28 stores the evaluation result ER of the GTC evaluation for each partial region performed by the evaluation unit 27. For example, the user can read out the evaluation result ER through the input device 32 after the examination of the subject and consider the risk of cancer in the breast of the subject based on the evaluation result ER. As the evaluation result memory 28, for example, recording media such as a flash memory, an HDD, an SSD, an FD, an MO disc, an MT, a RAM, a CD, a DVD, an SD card, and an USB memory can be used.

The notification unit 29 issues notification to the user in a case in which the evaluation result ER of the GTC evaluation performed by the evaluation unit 27 corresponding to any of the plurality of partial regions exceeds a predetermined threshold value. In a case in which the evaluation result ER of the GTC evaluation performed by the evaluation unit 27 is the ratio of the GTC region R1 in the mammary gland region M, the notification unit 29 issues the notification to the user in a case in which the value of the ratio of the GTC region R1 exceeds the predetermined threshold value. In addition, in a case in which the evaluation result ER of the GTC evaluation performed by the evaluation unit 27 is the category of the GTC region R1, the notification unit 29 notifies the user that the category representing that the atrophy of the lobule has not progressed as compared to the predetermined category is output.

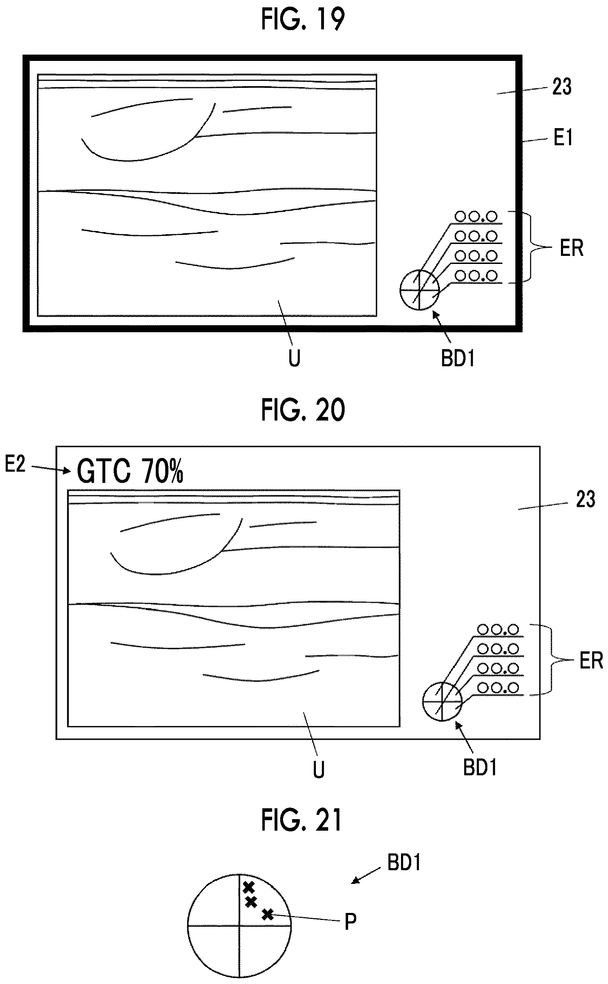

The notification unit 29 can issue the notification to the user by, for example, highlighting a specific portion of a display screen of the monitor 23 as shown in Fig. 19. Fig. 19 shows an example in which the user is notified by highlighting a frame line E1 of the display region of the monitor 23. In addition, for example, as shown in Fig. 20, the notification unit 29 can also issue the notification to the user by displaying a numerical value E2 representing the ratio of the GTC region R1 exceeding the predetermined threshold value on the monitor 23.

In addition, in a case in which the ultrasonic diagnostic apparatus comprises a position sensor (not shown) attached to the ultrasonic probe 1, the notification unit 29 can issue the notification to the user by displaying, for example, a position P where the ultrasonic image U in which the evaluation result ER exceeds the threshold value is captured on the breast schematic diagram BD1 in a superimposed manner on the monitor 23 based on a position of the ultrasonic probe 1 detected by the position sensor, as shown in Fig. 21. The position sensor can include at least one of, for example, an acceleration sensor, a gyro sensor, a magnetic sensor, or a global positioning system (GPS) sensor. The user can easily ascertain that the risk of cancer is high in the breast of the subject by confirming the notification issued by the notification unit 29.

The monitor 23 displays the ultrasonic image U under the control of the display control unit 22, and includes, for example, a display device such as a liquid crystal display (LCD) or an organic electroluminescence display (organic EL display).

The body control unit 31 controls each unit of the apparatus body 2 and the transmission-and-reception circuit 12 of the ultrasonic probe 1 based on a control program or the like, which is stored in advance.

The input device 32 is an input device used by the user to perform an input operation, and is configured by, for example, a device such as a keyboard, a mouse, a trackball, a touchpad, and a touch sensor disposed in a state of being superimposed on the monitor 23.

The processor 34 including the image generation unit 21, the display control unit 22, the mammary gland region extraction unit 25, the GTC region extraction unit 26, the evaluation unit 27, the notification unit 29, and the body control unit 31 is configured by a central processing unit (CPU) and a control program for causing the CPU to execute various kinds of processing, but the processor 34 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 21, the display control unit 22, the mammary gland region extraction unit 25, the GTC region extraction unit 26, the evaluation unit 27, the notification unit 29, and the body control unit 31 of the processor 34 can also be configured by being integrated partially or entirely into one CPU or the like.

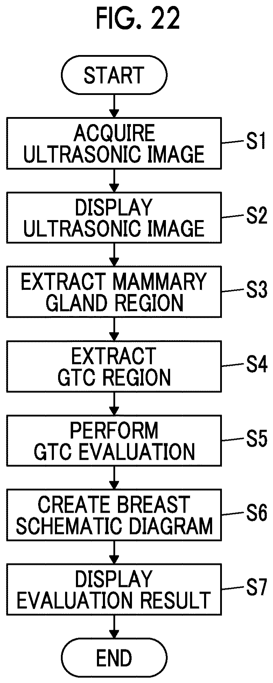

Next, an operation of the ultrasonic diagnostic apparatus according to the embodiment will be described with reference to a flowchart shown in Fig. 22.

First, in step S1, the breast of the subject is imaged by using the ultrasonic probe 1, and the ultrasonic image U is acquired. In this case, under the control of the body control unit 31, the transmission and reception of the ultrasonic waves from the plurality of transducers of the transducer array 11 are started in accordance with the drive signal from the pulser 13 of the transmission-and-reception circuit 12 of the ultrasonic probe 1, the ultrasonic echo from the inside of the breast of the subject is received by the plurality of transducers of the transducer array 11, the reception signal which is an analog signal is output to the amplifying unit 14 and is amplified by the amplifying unit 14, and the amplified reception signal is AD-converted by the AD conversion unit 15 to acquire the reception data.

The reception focus processing is performed on the reception data by the beam former 16, and the sound ray signal generated by the reception focus processing is transmitted to the image generation unit 21 of the apparatus body 2, and as a result, the ultrasonic image U representing the tomographic image information of the breast of the subject is generated by the image generation unit 21. In this case, the signal processing unit 41 of the image generation unit 21 performs the correction of the attenuation in accordance with the depth of the reflection position of the ultrasonic wave and the envelope detection processing on the sound ray signal, the DSC 42 performs the conversion into the image signal in accordance with the normal television signal scanning method, and the image processing unit 43 performs various types of necessary image processing such as gradation processing.

Next, in step S2, the ultrasonic image U generated by the image generation unit 21 is displayed on the monitor 23 via the display control unit 22, and is stored in the image memory 24.

In a case of acquiring the ultrasonic image U, the transmission intensity of the ultrasonic wave and the depth range of the ultrasonic image U displayed on the monitor 23 are adjusted under the control of the body control unit 31 such that the entire breast of the subject, that is, for example, the breast region BR of the subject shown in Fig. 4 or Fig. 5 is within the screen.

In subsequent step S3, the mammary gland region extraction unit 25 detects the breast region BR of the subject from the ultrasonic image U acquired in step S1, and extracts the mammary gland region M from the detected breast region BR. The mammary gland region extraction unit 25 can perform the image recognition using at least one of template matching, an image analysis technique using a feature value, such as AdaBoost, SVM, or SIFT, or a determination model that has been trained using a machine learning technique such as deep learning, in order to detect the breast region BR and to extract the mammary gland region M, for example.

In step S4, the GTC region extraction unit 26 extracts the GTC region R1 from the mammary gland region M of the subject extracted in step S3. The GTC region extraction unit 26 can distinguish between the GTC region R1 and the edematous stroma R2 in the mammary gland region M by, for example, binarizing the mammary gland region M of the ultrasonic image U using the brightness threshold value Thb, and can extract the GTC region R1. In addition, the GTC region extraction unit 26 can extract the GTC region R1 using the trained model that has learned the GTC region R1 (segmentation) in the mammary gland region M in the training ultrasonic image in which the breast is imaged, in machine learning such as deep learning.

In step S5, the evaluation unit 27 calculates the ratio of the GTC region R1 extracted in step S4 in the mammary gland region M extracted in step S3, and performs the GTC evaluation based on the calculated ratio of the GTC region R1. In this case, the evaluation unit 27 divides the breast of the subject into the plurality of partial regions and performs the GTC evaluation for each of the divided partial regions.

The evaluation unit 27 can calculate the ratio of the GTC region in the partial region, for example, by the ratio of the sum of the number of pixels occupied by all the GTC regions R1 present in the mammary gland region M to the number of pixels occupied by the mammary gland region M in the ultrasonic image U. The evaluation unit 27 can use, for example, the ratio of the GTC region R1 for each partial region calculated as described above as the evaluation result ER.

In addition, for example, the evaluation unit 27 can determine the category of the GTC region R1 based on the ratio of the GTC region R1 in the mammary gland region M and use the category as the evaluation result ER. In this case, the evaluation unit 27 can output any one of a plurality of predetermined categories, for example, the two categories of Low and High, or the four categories of Minimal, Mild, Moderate, and Marked, as the category of the GTC region R1.

In step S6, the schematic diagram creation unit 30 creates the breast schematic diagram BD1 representing the breast divided into the plurality of partial regions, for example, as shown in Fig. 6.

Finally, in step S7, for example, as shown in Fig. 14, the display control unit 22 displays the evaluation result ER of the GTC evaluation performed in step S5 on the monitor 23 in association with the partial region corresponding to the position where the ultrasonic image U is captured among the plurality of partial regions in the breast schematic diagram BD1 created in step S6. The user can confirm the evaluation result ER of the GTC evaluation displayed in association with the partial region of the breast schematic diagram BD1, to consider the risk of cancer in detail for each partial region.

In a case in which the processing of step S7 is completed in this manner, the operation of the ultrasonic diagnostic apparatus according to the flowchart of Fig. 22 is completed.

As described above, with the ultrasonic diagnostic apparatus according to the embodiment, the mammary gland region extraction unit 25 extracts the mammary gland region M from the ultrasonic image U, the GTC region extraction unit 26 extracts the GTC region R1 from the mammary gland region M, the evaluation unit 27 performs the GTC evaluation based on the ratio of the GTC region R1 in the mammary gland region M, the schematic diagram creation unit 30 creates the breast schematic diagram BD1 representing the breast divided into the plurality of partial regions, and the display control unit 22 displays the evaluation result ER of the GTC evaluation performed by the evaluation unit 27 on the monitor 23 in association with the partial region corresponding to the position where the ultrasonic image U is captured among the plurality of partial regions in the breast schematic diagram BD1, so that the user can confirm the evaluation result ER of the GTC evaluation displayed for each of the plurality of partial regions on the monitor 23, to consider the risk of cancer in detail for each of the plurality of partial regions.

In addition, a case has been described in which the transmission-and-reception circuit 12 is provided in the ultrasonic probe 1, but the transmission-and-reception circuit 12 may be provided in the apparatus body 2.

Furthermore, a case has been described in which the image generation unit 21 is provided in the apparatus body 2, but the image generation unit 21 may be provided in the ultrasonic probe 1.

Moreover, the apparatus body 2 may be a so-called stationary type, a portable type that is easy to carry, or a so-called handheld type that is configured by, for example, a smartphone or a tablet type computer. As described above, the type of the device constituting the apparatus body 2 is not particularly limited.

In addition, a case has been described in which the ultrasonic probe 1 and the apparatus body 2 are connected to each other in a wired manner, but the ultrasonic probe 1 and the apparatus body 2 may be connected to each other in a wireless manner.

In addition, although it has been described that the mammary gland region extraction unit 25 extracts the mammary gland region M from the ultrasonic image U, the mammary gland region extraction unit 25 may not be able to extract the mammary gland region M due to some reason, for example, in a case in which the ultrasonic image U is unclear. In this case, the GTC region extraction unit 26 can perform processing of extracting the GTC region R1 only for the ultrasonic image U of the frame in which the mammary gland region M is normally extracted by the mammary gland region extraction unit 25, among the ultrasonic images U continuously generated by the image generation unit 21. As a result, the evaluation unit 27 can calculate the ratio of the GTC region R1 with high accuracy and perform the GTC evaluation with high accuracy.

In addition, although it has been described that the evaluation unit 27 calculates the ratio of the GTC region R1 based on the mammary gland region M and the GTC region R1 extracted from the ultrasonic image U of one frame to perform the GTC evaluation, the evaluation unit 27 can also calculate the ratio of the GTC region R1 based on the mammary gland region M and the GTC region R1 extracted from the ultrasonic images U of a plurality of frames for each partial region to perform the GTC evaluation.

In this case, for example, the evaluation unit 27 can calculate the ratio of the GTC region R1 in the mammary gland region M for each of the ultrasonic images U of a predetermined number of frames, calculate a final ratio of the GTC region R1 using the average value, the median value, the most frequent value, or the maximum value of the values of the ratios, and perform the GTC evaluation for each partial region based on the final ratio of the GTC region R1. In this case, the evaluation unit 27 can calculate the final ratio of the GTC region R1 using the values of the plurality of ratios excluding outliers among the ratios of the GTC region R1 in the mammary gland region M for each of the ultrasonic images U of the predetermined number of frames. The outlier means a value in which a difference between a plurality of values is larger than a predetermined difference threshold value. In addition, the number of frames of the ultrasonic image U used for the GTC evaluation can be determined in advance, for example, by an input operation performed by the user through the input device 32.

In this case, for example, the evaluation unit 27 can calculate the similarity between the ultrasonic images U of the plurality of frames, calculate the ratio of the GTC region R1 based on the mammary gland region M and the GTC region R1 extracted from the ultrasonic image U of the frame in which the similarity is lower than a predetermined similarity threshold value, and perform the GTC evaluation.

The evaluation unit 27 can calculate the similarity between the ultrasonic images U of the plurality of frames using, for example, at least one of an image analysis technique using a feature value, such as AdaBoost, SVM, or SIFT, or the determination model that has been trained using a machine learning technique such as deep learning. In addition, the evaluation unit 27 can also calculate the similarity based on, for example, a magnitude of a difference in pixel values between the ultrasonic images U, a difference in distribution of the pixel values between the ultrasonic images U, or a difference in ratio of the mammary gland region M between the ultrasonic images U. Further, the evaluation unit 27 can determine that the ultrasonic images U of the consecutive frames are similar to each other, thins out the ultrasonic images U of the plurality of consecutive frames at regular intervals, and then calculate the similarity between the ultrasonic images U of the remaining plurality of frames.

In this way, by performing the GTC evaluation for each partial region based on the mammary gland region M and the GTC region R1 extracted from the ultrasonic images U that are not similar to each other, it is possible to prevent the ratio of the GTC region R1 calculated for the ultrasonic image U of each frame from being biased to similar values, and to perform the GTC evaluation with higher accuracy.

In addition, the evaluation unit 27 can also perform the GTC evaluation for each partial region based on the ultrasonic image U of the latest frame or the frame designated by the user via the input device 32 and the mammary gland region M and the GTC region R1 extracted from the ultrasonic images U of a predetermined number of frames consecutive to the frame in the past.

In addition, although it has been described that the evaluation unit 27 calculates the ratio of the GTC region R1 in the mammary gland region M and determines the category of the GTC region R1 based on the calculated ratio, for example, the GTC evaluation can also be performed by determining the category of the GTC region R1 based on the mammary gland region M extracted by the mammary gland region extraction unit 25 by using a machine learning method. In this case, the evaluation unit 27 can output the category of the GTC region R1 in the mammary gland region M by inputting the mammary gland region M extracted by the mammary gland region extraction unit 25 to a trained model that has learned the ultrasonic image U of the mammary gland region M of the subject and the category of the GTC region R1 in the mammary gland region M.

In addition, the evaluation unit 27 can output the category of the GTC region R1 in the mammary gland region M shown in the ultrasonic image U by inputting the ultrasonic image U read out from the image memory 24 to the trained model that has learned, for example, the ultrasonic image U in which the mammary gland region M is shown and the category of the GTC region R1 in the mammary gland region M.

Further, although it has been described that the evaluation result ER of the GTC evaluation output by the evaluation unit 27 is stored in the evaluation result memory 28, the evaluation result ER can also be stored in association with the ultrasonic image U used for the GTC evaluation.

In addition, the apparatus body 2 can also comprise a transmission circuit (not shown) that transmits the evaluation result ER of the GTC evaluation for each partial region output by the evaluation unit 27 to an external server device (not shown) such as an examination information management system such as a so-called electronic medical record, a report system that creates a report using a medical image, and a picture archiving and communication system (PACS). In a case of transmitting the evaluation result ER to the external server device or the like, for example, a protocol, such as hypertext transfer protocol (HTTP), hypertext transfer protocol secure (HTTPS), file transfer protocol (FTP), health level seven (HL7), or digital imaging and communications in medicine (DICOM), can be used.

In addition, although it has been described that the display control unit 22 displays the evaluation result ER of the GTC evaluation on the monitor 23 in association with the plurality of partial regions of the breast schematic diagram BD1, for example, the evaluation result ER can be displayed in association with only a partial region designated by the user via the input device 32 among the plurality of partial regions of the breast schematic diagram BD1. As a result, the user can consider the risk of cancer in detail only for the partial region of interest.

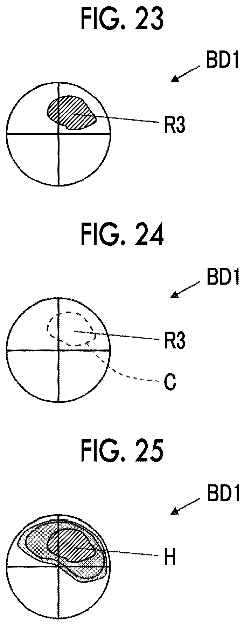

It is generally known that the risk of occurrence of breast cancer is higher as the mammary gland volume in the breast of the subject is larger. Therefore, for example, the mammary gland region extraction unit 25 can calculate the mammary gland volume in the extracted mammary gland region M. In this case, for example, the display control unit 22 can provide a predetermined mammary gland volume threshold value for the mammary gland volume and display an excessive mammary gland volume region R3, in which the mammary gland volume is larger than the mammary gland volume threshold value, in a superimposed manner on the breast schematic diagram BD1, as shown in Fig. 23. Fig. 23 shows an example in which a color different from the surrounding color is given to the excessive mammary gland volume region R3.

In addition, for example, as shown in Fig. 24, the display control unit 22 can also display a contour line C of the excessive mammary gland volume region R3 in a superimposed manner on the breast schematic diagram BD1. In addition, for example, as shown in Fig. 25, the display control unit 22 can also display a so-called heat map H showing a distribution of the mammary gland volume based on the mammary gland volume calculated by the mammary gland region extraction unit 25 in a superimposed manner on the breast schematic diagram BD1.

The user can more specifically consider the risk of cancer in the breast of the subject by confirming the excessive mammary gland volume region R3 or the heat map H displayed in a superimposed manner on the breast schematic diagram BD1 in this manner.

### Explanation of References

1: ultrasonic probe
2: apparatus body
11: transducer array
12: transmission-and-reception circuit
13: pulser
14: amplifying unit
15: AD conversion unit
16: beam former
21: image generation unit
22: display control unit
23: monitor
24: image memory
25: mammary gland region extraction unit
26: GTC region extraction unit
27: evaluation unit
28: evaluation result memory
29: notification unit
30: schematic diagram creation unit
31: body control unit
32: input device
33: image acquisition unit
34: processor
41: signal processing unit
42: DSC
43: image processing unit
BD1 to BD7: breast schematic diagram
BR: breast region
C: contour line
E1: frame line
E2: numerical value
ER: evaluation result
H: heat map
L1: front boundary line
L2: rear boundary line
M: mammary gland region
P: position
R1: GTC region
R2: edematous stroma
R3: excessive mammary gland volume region
S: skin
T: pectoralis major
U: ultrasonic image

## Claims

1. An ultrasonic diagnostic apparatus comprising:
an image acquisition unit that acquires an ultrasonic image in which a mammary gland region of a subject is imaged;
a monitor that displays the ultrasonic image;
a mammary gland region extraction unit that extracts the mammary gland region from the ultrasonic image;
an evaluation unit that performs a glandular tissue component evaluation based on the mammary gland region extracted by the mammary gland region extraction unit;
a schematic diagram creation unit that creates a breast schematic diagram showing a breast divided into a plurality of partial regions; and
a display control unit that displays an evaluation result of the glandular tissue component evaluation performed by the evaluation unit on the monitor in association with a partial region, which corresponds to a position where the ultrasonic image is captured, among the plurality of partial regions in the breast schematic diagram.

2. The ultrasonic diagnostic apparatus according to claim 1, further comprising:
a glandular tissue component region extraction unit that extracts a glandular tissue component region from the mammary gland region extracted by the mammary gland region extraction unit,
wherein the evaluation unit performs the glandular tissue component evaluation based on a ratio of the glandular tissue component region in the mammary gland region.

3. The ultrasonic diagnostic apparatus according to claim 2,
wherein the evaluation unit displays the ratio of the glandular tissue component region in the mammary gland region on the monitor as the evaluation result.

4. The ultrasonic diagnostic apparatus according to claim 2,
wherein the evaluation unit determines a category of the glandular tissue component region based on the ratio of the glandular tissue component region in the mammary gland region, and displays the category on the monitor as the evaluation result.

5. The ultrasonic diagnostic apparatus according to claim 1,
wherein the evaluation unit performs the glandular tissue component evaluation using a determination model that has been trained through machine learning.

6. The ultrasonic diagnostic apparatus according to claim 1,
wherein the image acquisition unit acquires a plurality of the ultrasonic images at positions corresponding to the same partial region, and
the evaluation unit determines the evaluation result in the partial region based on a plurality of the evaluation results acquired by performing the glandular tissue component evaluation on each of the plurality of ultrasonic images.

7. The ultrasonic diagnostic apparatus according to claim 6,
wherein the evaluation unit determines the evaluation result for a specific ultrasonic image by taking into account the plurality of evaluation results in the ultrasonic images of a predetermined number of frames immediately before the specific ultrasonic image.

8. The ultrasonic diagnostic apparatus according to claim 2,
wherein the glandular tissue component region extraction unit extracts the glandular tissue component region only for the ultrasonic image from which the mammary gland region is extracted by the mammary gland region extraction unit.

9. The ultrasonic diagnostic apparatus according to claim 1, further comprising:
a notification unit that issues notification to a user in a case in which the evaluation result of the glandular tissue component evaluation performed by the evaluation unit exceeds a predetermined threshold value.

10. The ultrasonic diagnostic apparatus according to claim 9,
wherein the notification unit displays a position where the ultrasonic image in which the evaluation result exceeds the threshold value is captured, on the breast schematic diagram.

11. The ultrasonic diagnostic apparatus according to claim 9,
wherein the notification unit highlights a specific portion of a display screen of the monitor.

12. A method of controlling an ultrasonic diagnostic apparatus, the method comprising:
acquiring an ultrasonic image in which a mammary gland region of a subject is imaged;
displaying the ultrasonic image on a monitor;
extracting the mammary gland region from the ultrasonic image;
performing a glandular tissue component evaluation based on the extracted mammary gland region;
creating a breast schematic diagram showing a breast divided into a plurality of partial regions; and
displaying an evaluation result of the glandular tissue component evaluation on the monitor in association with a partial region, which corresponds to a position where the ultrasonic image is captured, among the plurality of partial regions in the breast schematic diagram.
